# EUROPEAN PATENT APPLICATION

(11) **EP 1 449 536 A1**
(43) Date of publication of application: **25.08.2004**
(21) Application number: 03003375.7
(22) Date of filing: 14.02.2003
(51) Int. Cl.: A61K 38/17, A61P 27/00, A61P 25/28, A61P 9/10, A61P 17/02

(54) **Prion proteins (PrP) as therapeutic agents for the treatment of AP-1 associated diseases**

(71) Applicant: Prionics AG, CH-8952 Schlieren (CH)
(72) Inventor: Frigg, Rico, 8001 Zürich (CH)
(74) Representative: Emmel, Thomas, Dipl.-Biol., Dr.

(57) **Abstract**

Method of preparing a therapeutic agent for the inhibition of AP-1 induced processes, characterised in that PrP or modifications thereof are being included in the agent as effective component.

## Description

### Background

The prion protein (PrP) is a cell membrane bound and secreted glycoprotein which is highly conserved among species and therefore hypothesized to exhibit important biological functions. However, up to date the physiological roles of the cellular prion protein (PrPc) have remained greatly elusive. PrPc is expressed in many cell types of vertebrates, predominantly though in nervous tissue. Gene knockout studies in mice showed that PrPc is basically dispensable for the development and maintenance of a mammalian organism. N-terminally located octapeptide repeat motifs of the PrP molecule have been implicated in copper metabolism and have been implicated in superoxide dismutase-like activity. In vitro and ex vivo studies gave rise to the assumption that PrPc might be involved in protective mechanisms against conditions eliciting cellular stress and eventually inducing cell death. Experiments comparing the effect of metabolic and/or oxidative stress on wild-type cells and on cells lacking the PrPc (PrP-knockout, Prnp0/0) showed that Prnp0/0 cells are less resistant to these adverse conditions.

Apoptosis constitutes a mode of cellular death with characteristic morphological features, orchestrated by distinct molecular signalling cascades, and modulated by pro- and anti-apoptotic factors. Another appearance of cell death in biological systems is referred to as necrosis, which in general is associated with inflammation and collateral damage of cells and tissue. However, in affected biological systems such as tissues or organs both forms of cell death can be present. Since apoptosis is an energy-requiring event, it is hypothesized that necrosis occurs in case of a lack of sufficient metabolic resources.

In WO 0249662 a method of modulating apoptosis in cultured cells is disclosed. The method makes use of the fact that PrP is able to abrogate a pro-apoptotic effect induced by over-expression of Bax, a pro-apoptotic member of the Bcl-2 family and associated with the mitochondrial apoptotic pathway. In particular, this effect of the PrP was assigned to the presence of the N-terminal octapeptide region of the molecule.

Up to date, there are however no data affirming a putative protective function of PrP in vivo. We have, for the first time, found a potential protective/anti-apoptotic means of the prion protein in vivo by taking advantage of a model system of acute neuronal degeneration, namely of light-induced retinal degeneration. In this model photoreceptor cells undergo apoptotic death when challenged with light doses exceeding a certain threshold. The suicidal program of light-triggered photoreceptor cell death involves up-regulation of the activator protein 1 (AP-1) complex and of caspase-1. Proteins of the Bcl-2 family are not involved in this pathway. We demonstrate that peripheral application of bacterially produced, un-glycosylated prion protein confers protection from light toxicity to wild-type mice. Moreover, genetically engineered mice overexpressing PrPc (tga20) are highly protected from light toxicity. These mice did not show caspase-1 activation, whereas AP-1 induction was observed, suggesting that PrP acts between the two steps of this apoptotic pathway.

The activator protein 1 (AP-1) complex is a dimeric complex consisting of members of the Fos and Jun protein families. AP-1 complexes exist as homo- or heterodimers and act as nuclear third messengers. They are involved in a wealth of signal transduction pathways by way of induction, suppression, or modulation of the expression of target genes. Increased AP-1 DNA-binding activity can be observed following various pathological stimuli such as light-induced toxicity in retinal cells, infarction-induced ischemia in brain tissue and heart tissue, or in seizure-induced excitotoxic damage to limbic neurons.

Caspases are end stage effectors of the various apoptotic cascades and activated specifically in apoptotic cells. They are cysteine proteases which degrade proteins and peptides by way of cleaving them within distinct aminoacid motifs, leading to disintegration of cells with characteristic morphological features. The different caspases are homologous to each other and highly conserved among species. Because caspases can induce most of the observable changes that characterize apoptosis, they can be considered the central executioners of apoptotic pathways. This notion is solidified by the finding that inhibitors of caspases can slow or even prevent apoptosis. Thus, inhibition of caspases/caspase activation offers a very promising approach to interfere with conditions associated with acute disturbances of metabolic homeostasis of cells, tissues, and organs with induction of apoptotic cascades.

In addition, caspase-1, also called Il-1 beta converting enzyme, has a function in cytokine maturation. Il-1 beta and Il-18 are cleaved and thereby activated by caspase-1. These cytokines harbour key roles in many inflammatory processes such as rheumatoid arthritis, or inflammatory bowel disease. Caspase-1 knockout mice as well as mice treated with an inhibitor of caspase-1 are protected against experimental mucosal inflammation. Thus, inhibition of caspases/caspase activation could provide a new and promising approach to treat acute or chronic inflammatory diseases.

It is a major objective of the present invention to provide a method of preparing a therapeutic agent for the inhibition of AP-1 induced processes, which can result in apoptosis or acute inflammation. It is a further objective of the present invention to provide a method of influencing the regulation of AP-1 induced effectors in cells, tissues or organs. These objectives are met by the characterising features of claims 1 and 12.

### Brief summary of the invention

In general, the present invention is directed towards the application of PrP or modifications thereof as effective component in a therapeutic agent for the inhibition of AP-1 induced processes, like the activation of an enzyme of the caspase family. These processes may result in either apoptosis or acute or chronic inflammation.

The application of said therapeutic agent may thus contribute to the protection of vertebrate cells, tissues, and organs in vivo from conditions which for example generate metabolic stress, with subsequent activation of apoptotic machineries with caspase induction, in particular with caspase-1 induction.

The observation that peripherally administered, recombinant PrP is able to protect photoreceptor cells in vivo as illustrated in the present invention argues for mechanisms allowing the PrP to trespass the vasculature, more particular the blood-retina barrier, which in nature is similar to the blood-brain barrier, to unfold a protective means in cells which otherwise would die away. Thus, one particular aspect of the invention is that the cells, tissues and organs targeted by the therapeutic agent of the present invention are located distal to the vasculature by virtue of an endothelial barrier.

One particular aspect of the present invention is the use of PrP and modifications thereof for the preparation of a therapeutic agent for the protection of cells, tissues and organs from AP-1 induced processes, which otherwise would lead to acute degeneration, in particular to acute neuro-degeneration in vivo. These processes may be evoked by particular physical, chemical, or biochemical conditions. Non-limiting examples of such processes are light-induced retinal degeneration, ischemia-induced retinal degeneration, trauma-induced retinal degeneration, ischemia-induced cerebral degeneration, trauma-induced cerebral degeneration, seizure-induced neuronal cell death, ischemia-induced myocardial cell death.

Another aspect of the invention is the use of chemically, biochemically, or genetically modified PrPs, or fragments thereof retaining the anti-apoptotic activity and, of course, combinations thereof for the preparation of a therapeutic agent serving the above mentioned purposes.

A further aspect of the invention is that the said therapeutic agent is adapted for administration by subcutane, intramuscular or intravenous injection. It may moreover be adapted for oral or topical administration.

Another possible way of administration of said therapeutic agent is by overexpression in genetically modified cells. Said cells may have been implanted into a target organism before, or they may be used as stem cells form which a target organism is produced.

It is another aspect of the present invention to influence the regulation of AP-1 induced effectors in cells, tissues or organs by applying PrP or effective modifications thereof in a sufficient dosis. Said AP-1 influenced effector may for example be an enzyme of the caspase family, and it may be involved in cellular apoptosis or in inflammatory processes.

Another aspect of the invention is the use of chemically, biochemically, or genetically modified PrPs, or fragments thereof retaining the anti-apoptotic activity-and, of course, combinations thereof for the preparation of a therapeutic agent serving the above mentioned purposes.

### Description of the figures

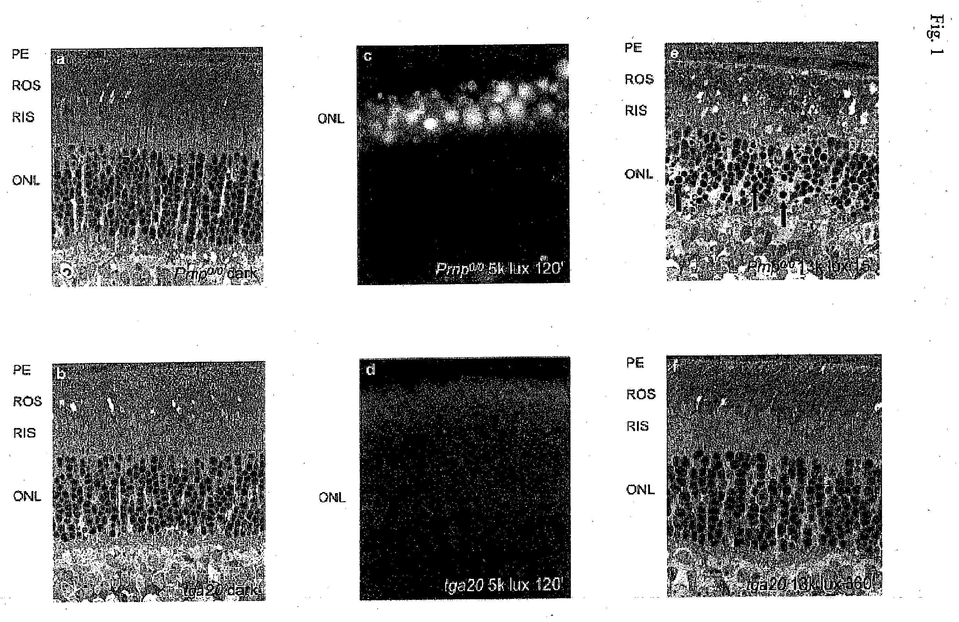

### Fig. 1: Strong protection of PrP-overexpressing mice against light-toxicity.

Histological examination showed normal retinal morphology of dark-adapted PrP-knockout (Prnp0/0) and PrP-overexpressing (tga20) control mice (Fig. la and b, respectively). Prnp0/0 mice exposed to 5000 lux of light intensity for 120 minutes exhibited retinal degeneration and photoreceptor apoptosis as shown by the positive TUNEL labelling of most nuclei in the outer nuclear layer (Fig. 1c). In contrast, retinal integrity was unaffected in tga20 mice and photoreceptor nuclei were TUNEL-negative (Fig. 1d). When challenged with even higher light intensity (13000 lux) Prnp0/0 photoreceptors displayed severe cellular disintegration with disruption of rod outer and inner segments as well as nuclear condensation within 15 minutes of exposure time (Fig. 1e). On the other hand, in tga20 mice retinal morphology was preserved and no photoreceptor demise was present even after exposure to 13000 lux for 360 minutes (Fig. 1f).

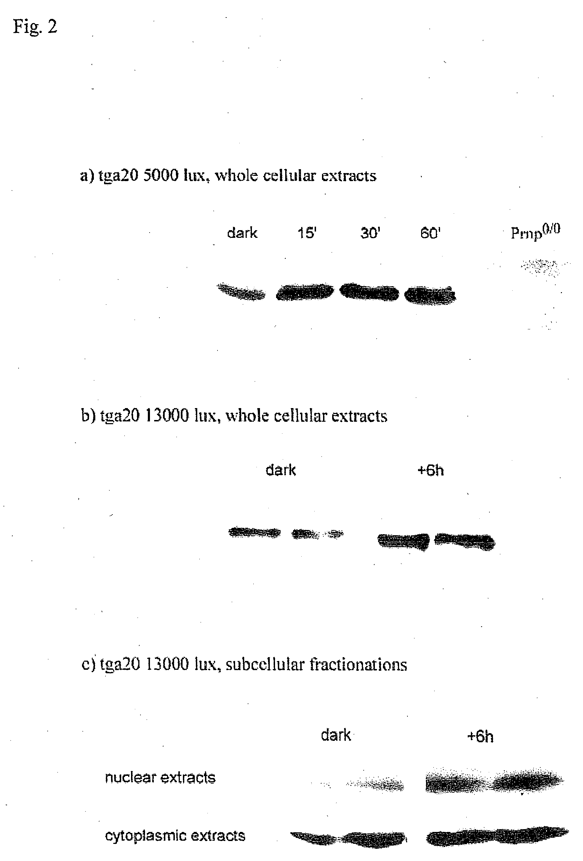

### Fig. 2: Regulation of PrP upon exposure to light stimulus.

Retinal light damage is mediated by apoptotic cell death as illustrated in Figure 1c by TUNEL staining. Assuming that PrPc would directly interfere with the apoptotic signalling pathway we analysed the fate of PrP as well as the activation of two key factors of light-induced photoreceptor apoptosis (AP-1 and caspase-1). Upon light exposure, PrPc was rapidly induced in tga20 mice, reaching its plateau within fifteen minutes after onset of light exposure (Fig 2a) and persisting at high levels for several hours after cessation of the stimulus (Fig. 2b). Subcellular fractionation of retinal cells revealed increased cytoplasmic and nuclear PrPc indicating nuclear accumulation (Fig. 2c), which is suggestive for nuclear sites of action of PrP in prevention of apoptosis.

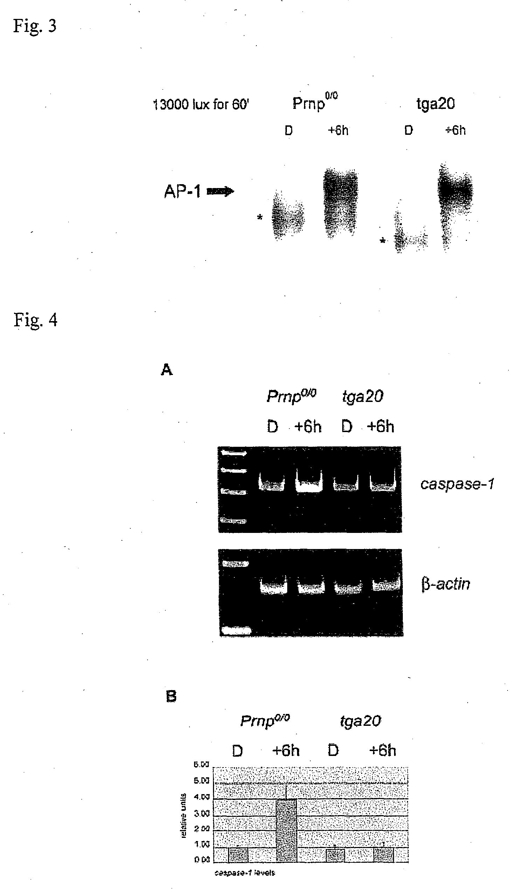

### Figs. 3, 4: Interference of PrP with apoptotic signalling cascade.

The apoptotic pathway in light-induced retinal degeneration involves AP-1 as well as caspase-1 induction. AP-1 DNA-binding activity of retinal nuclear extracts of Prnp0/0 and tga20 mice was measured by electrophoretic mobility shift assays. AP-1 activity was induced in both types of mice despite the lack of apoptosis in tga20 mice (Fig.3). The regulation of caspase-1 gene expression in light-exposed and non-exposed retinae of Prnp0/0 and tga20 mice was analysed by RT-PCR. As shown in Figure 4 caspase-1 mRNA levels are elevated in Prnp0/0 mice exposed to damaging light as opposed to tga20 mice exposed to the same light dose or non-exposed control mice. Caspase induction is considered a late stage event in various apoptotic pathways, which is in agreement with caspase-1 induction and morphological appearance of apoptotic photoreceptors in Prnp0/0 mice.

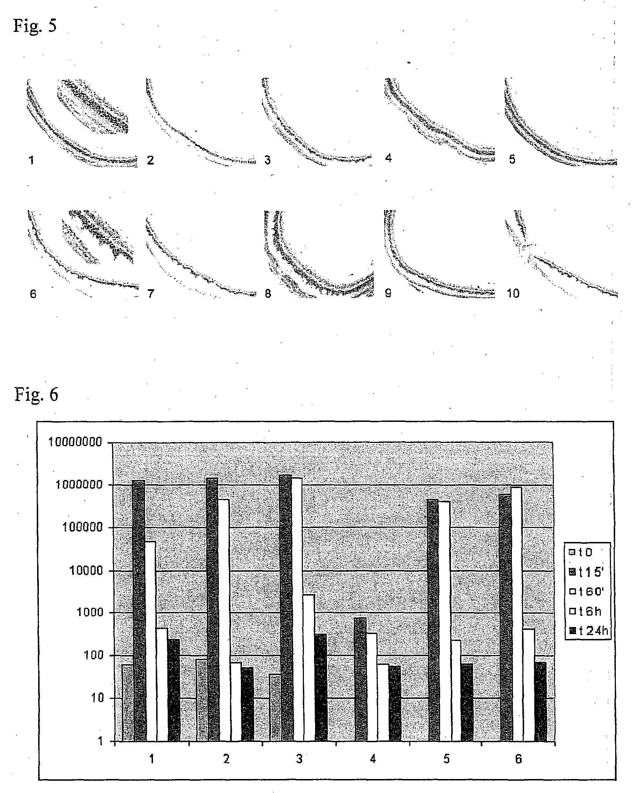

### Fig. 5: Protection of wild-type mice from light-damage by exogenous PrP.

Peripheral administration of recombinant PrP was able to protect mice from light-induced retinal damage. Balb/C wild-type mice were treated with recombinant bovine PrP by way of intraperitoneal injections. Experimental mice received doses of 300mg one hour prior to, one hour after and three hours after exposure to 5800 lux for 30 minutes (Fig. 5, 1-5). Control mice received water injections at the corresponding time points (Fig. 5, 6-10). Histological examination one day after light exposure showed that in treated mice photoreceptor damage was dramatically reduced as compared to vehicle-injected control animals (Fig. 5).

### Fig. 6: Metabolism of recombinant PrP after peripheral administration.

To study the uptake and metabolism of peripherally administered, bacterially-produced recombinant murine PrP, we measured serum levels of PrP in Prnp0/0 mice having received either a single intravenous (i.v.), or a single intraperitoneal (i.p.) injection of 100mg of recombinant, un-glycosylated murine PrP. Figure 6 shows serum levels (arbitrary units) of applied PrP at different time points, in individuals 1 to 3 after i.v. administration, in individuals 4 to 6 after i.p. administration. The figure shows that i.p. application is slightly less efficient than i.v. application and that levels of PrP in serum drop to marginally above background within six hours after administration. 24 hours after injection no PrP signal was detectable anymore.

### Methods

Mice and light exposure: For experiments offspring at the age of 7-10 weeks was used. Prnp0/0, tga20, and Balb/C wild-type mice were kept under a 12h:12h (6:00/6:00) light:dark cycle (60lux at bottom of cages). Prior to light exposure, animals were dark-adapted for 16h overnight. The pupils of Prnp0/0and tga20 animals were dilated under dim red light (Cyclogyl 1%, Alcon, Cham, Switzerland and Phenylephrine 5%, Ciba Vision, Niederwangen, Switzerland) and Prnp0/0and tga20 mice were exposed to diffuse white fluorescent light (TLD-36 W/965 tubes, Philips, Hamburg, Germany; UV-impermeable diffuser) for different time periods with an intensity of 5klux or 13klux in cages with a reflective interior. Balb/C mice were exposed to 5.8klux for 30 minutes. After light exposure, animals were analyzed immediately or after a period in darkness.

Injection of recombinant prion protein: Recombinantly produced, lyophilized un-glycosylated, bovine prion protein (rec boPrP) was dissolved in distilled water to a final concentration of 1mg/ml. In experimental mice injections of 300mg of rec boPrP were performed one hour prior to light exposure, one hour after light exposure and three hours after light exposure. In control mice 300ml of water were injected at the corresponding time points.

Light microscopy: Eyes were enucleated and fixed in 2.5% glutaraldehyde in 0.1M cacodylate buffer, pH 7.3 at 4°C overnight. For each eye, the superior central and the inferior central retina adjacent to the optic nerve were trimmed, washed in cacodylate buffer, incubated in osmium tetroxide for 1h, dehydrated in increasing ethanol concentrations and embedded in Epon 812. For light microscopy, sections (0.5mm) were prepared from the lower central retina (most affected in our light-damage model), counterstained with methylene blue and analyzed using an Axiophot microscope (Zeiss, Oberkochen, Germany).

TUNEL-assay: Eyes were fixed in 2% paraformaldehyde for 2h at 4°C followed by dehydration and paraffin embedding. TdT-mediated dUTP nick-end labeling (TUNEL) was performed with modifications using the "in situ cell death detection kit" (Boehringer Mannheim, Germany) on 5µm paraffin sections. DNA strand breaks were labeled with fluorescein and visualized with a FITC filter.

Western blotting: Whole retinal cellular homogenates were prepared by sonification of shock-frozen retinae in TBS. Nuclear and cytoplasmatic extracts were prepared as described previously. Briefly, one retina was homogenized in 400µl 10mM Hepes-KOH pH7.9, 1mM β-mercaptoethanol, 1mM DTT in the presence of protease inhibitors. After incubation on ice for 10min the homogenate was vortexed for 10sec and centrifuged. The pellet was resuspended in 50µl 20mM Hepes-KOH pH7.9, 25% glycerol, 420mM NaCl, 1.5mM MgCl2, 0.2mM EDTA, 1mM β-mercaptoethanol, 1 mM DTT in the presence of protease inhibitors and incubated on ice for 10min. Cellular debris was removed by centrifugation at 23'000g for 30min at 4°C. Protein concentrations were determined using the Bradford protein assay (Bio-Rad, Hercules, USA) with BSA as standard. Whole retinal homogenates, nuclear extracts, and cytoplasmatic extracts were deglycosylated for 2h at 37°C using PNGase F (New England Biolabs, Beverly, USA). Equal amounts of protein were subjected to SDS-Page, transferred to nitro-cellulose membranes and probed with a-prion protein monoclonal antibody 6H4 (Prionics AG, Schlieren, Switzerland).

Electrophoretic mobility shift assay (EMSA): EMSAs were performed as described previously. Briefly, the oligonucleotides coding for an AP-1 specific (5'-AAG CAT GAG TCA GAC AC-3') DNA binding sequence (TPA response element, TRE) were labelled using polynucleotide kinase (Boehringer Mannheim, Germany) and 32P-gATP (Hartmann Analytic GmbH, Braunschweig, Germany). For EMSA, 2-5µg (5ml) protein of nuclear extract were incubated on ice for 20min with 19µl 5mM MgCl2, 0.1mM EDTA, 0.75mM DTT, 7.5% glycerol, 0.05% NP-40 containing 24µg BSA and 2µg poly d(I-C) (Boehringer Mannheim, Germany). Radiolabeled oligonucleotide (1ml) was added and incubation was continued for another 20min. Protein/DNA complexes were resolved at 150V on a 1.5mm 6% polyacrylamide gel using 0.25x TBE buffer and visualized on X-ray film.

RNA isolation, cDNA synthesis, and PCR: Retinae were removed through a slit in the cornea and immediately frozen in liquid nitrogen. Retinae were stored at -70 °C until RNA preparation. Total retinal RNA was prepared using the RNeasy RNA isolation kit (Qiagen, Hilden, Germany). Reverse transcription was performed on 400 ng of total retinal RNA using oligo(dT) and M-MLV reverse transcriptase (Promega, Madison, USA). cDNAs corresponding to 10 ng of total RNA were amplified with primers specific for b-actin (see below). Amplification products were quantified on a PhosphorImager (Fuji, Tokyo, Japan) for standardization. Standardized cDNAs corresponding to 10 to 20 ng of total RNA were amplified by PCR using the following primer pairs and cycle numbers: b-actin: 24 cycles; up: 5'-CAA CGG CTC CGG CAT GTG C-3'; down: 5'-CTC TTG CTC TGG GCC TCG-3'. Caspase-1: 34 cycles; up: 5'-GAG AAG AGA GTG CTG AAT CAG-3'; down: 5'-CAA GAC GTG TAC GAG TGG TTG-3'. Downstream primers in all amplification reactions were 32P-end labeled. Amplification products were resolved on a 6% polyacrylamide gel and stained with ethidium bromide. Products were quantified on a PhosphorImager (Fuji).

## Claims

1. Method of preparing a therapeutic agent for the inhibition of AP-1 induced processes, **characterised in that** PrP or modifications thereof are being included in the agent as effective component.

2. Method according to claim 1, **characterised in that** said AP-1 induced process is the activation of an enzyme of the caspase family.

3. Method according to claim 1 or 2, **characterised in that** said AP-1 induced process is an apoptotic process.

4. Method according to claim 1 or 2, **characterised in that** said AP-1 induced process is an inflammatory process.

5. Method according to claim 1 or 2, **characterised in that** said AP-1 induced processes take place in cells, tissues and organs of a vertebrate, mammal or human.

6. Method according to claim 5, **characterised in that** said cells, tissues and organs are located distal to the vasculature by virtue of an endothelial barrier.

7. Method according to claim 1 or 2, **characterised in that** said AP-1 induced processes are:
(a) retinal degeneration due to light exposure, ischemia, trauma, optic nerve crush or optic nerve inflammation,
(b) cerebral degeneration due to ischemia, trauma, seizure or inflammation,
(c) spinal chord degeneration due to trauma or ischemia,
(d) myocardial degeneration due to ischemia, intestinal inflammation, arthritic inflammation, or
(e) UV-mediated skin lesioning.

8. Method according to claim 1 or 2, **characterised in that** said modifications consist of chemically, biochemically or genetically modified PrPs, or fragments or combinations thereof.

9. Method according to claim 1 or 2, **characterised in that** said therapeutic agent is adapted for administration by subcutane, intramuscular or intravenous injection.

10. Method according to claim 1 or 2, **characterised in that** said therapeutic agent is adapted for oral administration.

11. Method according to claim 1 or 2, **characterised in that** said therapeutic agent is adapted for topical administration.

12. Method of influencing the regulation of AP-1 induced effectors in cells, tissues or organs, **characterised by** the application of PrP or effective modifications thereof in a sufficient dosis.

13. Method according to claim 12, **characterised in that** the influenced effector is an enzyme of the caspase family.

14. Method according to claim 12, **characterised in that** the influenced effectors are involved in cellular apoptosis.

15. Method according to claim 12, **characterised in that** the influenced effectors are involved in inflammatory processes.

16. Method according to claim 12, **characterised in that** said modifications consist of chemically, biochemically or genetically modified PrPs, or fragments or combinations thereof.
